Europäisches Patentamt

(19) European Patent Office

Office européen des brevets

(11) Numéro de publication : **0 299 836 B1**

(12) # FASCICULE DE BREVET EUROPEEN

(45) Date de publication du fascicule du brevet :
**11.12.91 Bulletin 91/50**

(51) Int. Cl.$^5$ : **A61F 9/00, G02B 27/00**

(21) Numéro de dépôt : **88401691.6**

(22) Date de dépôt : **30.06.88**

(54) **Système optique et appareil chirurgical comportant ledit système.**

(30) Priorité : **08.07.87 FR 8709703**

(43) Date de publication de la demande :
**18.01.89 Bulletin 89/03**

(45) Mention de la délivrance du brevet :
**11.12.91 Bulletin 91/50**

(84) Etats contractants désignés :
**AT BE CH DE ES GB GR IT LI LU NL SE**

(56) Documents cités :
**EP-A- 0 191 688**
**EP-A- 0 207 648**
**EP-A- 0 224 322**
**US-A- 3 930 504**
**US-A- 4 397 310**
**PATENT ABSTRACTS OF JAPAN, vol. 7, no.
188 (P-217)[1333], 17 août 1983, page 58 P 217;
& JP-A-58 88 608 (TOUKIYOU KOSUMOSU
DENKI K.K.) 26-05-1983**

(73) Titulaire : **Azema, Alain**
**28 avenue des Arènes**
**F-06000 Nice (FR)**

Titulaire : **Arneodo, Jacques**
**55 Promenade des Anglais**
**F-06000 Nice (FR)**
Titulaire : **Botineau, Jean**
**Ile Verte 11 Place des Genèvriers**
**F-06560 Valbonne (FR)**
Titulaire : **Crozafon, Philippe**
**55, Promenade des Anglais**
**F-06000 Nice (FR)**
Titulaire : **Moulin, Gérard**
**4 Place Méjane Sophia Antipolis**
**F-06560 Valbonne (FR)**

(72) Inventeur : **Azema, Alain**
**28 avenue des Arènes**
**F-06000 Nice (FR)**
Inventeur : **Moulin, Gérard**
**167 avenue Maréchal Lyautey**
**F-06000 Nice (FR)**
Inventeur : **Botineau, Jean**
**5 avenue Chateaubriand**
**F-06000 Nice (FR)**

(74) Mandataire : **Bonnetat, Christian et al**
**CABINET BONNETAT 23, Rue de Léningrad**
**F-75008 Paris (FR)**

## Description

La présente invention concerne un système optique destiné à être disposé entre une source susceptible d'émettre un faisceau de lumière et une cible.

L'invention s'applique plus particulièrement, mais non exclusivement, à des appareils chirurgicaux destinés à modifier la courbure de la cornée, tels que ceux décrits dans les documents EP-A-0191688 et EP-A-0207648.

Parmi les différentes techniques connues de traitement chirurgical de certaines affections, comme la myopie et l'hypermétropie, par modification de la courbure de la cornée, l'élimination de matière cornéenne par photodécomposition à l'aide d'un faisceau de lumière ultraviolette produit par une source laser, en particulier un laser à excimère, est la technique la plus récente à avoir été envisagée.

Un système optique, comportant notamment une optique de focalisation, est disposé entre la source laser et l'oeil, lequel système dirige le faisceau sur la zone de cornée à éliminer et forme une tache lumineuse sur une partie de ladite zone, ladite tache étant susceptible d'explorer la totalité de ladite zone de cornée.

Cependant, du fait de caractéristiques qui lui sont inhérentes, le faisceau engendré par la source laser n'est pas toujours stigmatique, c'est-à-dire qu'il présente parfois, au foyer image d'une optique de focalisation, une section de forme oblongue, et non une section, "ponctuelle" ou quasi-ponctuelle, circulaire. Ainsi, une tache lumineuse, gardant une symétrie de révolution, c'est-à-dire qui reste circulaire ou quasiment circulaire, ne peut pas être obtenue sur la cible, en l'occurrence un oeil, lorsque ladite cible est déplacée relativement à ladite optique dans l'espace image de cette dernière, déplacement relatif qui est nécessaire pour permettre l'exploration par ladite tache lumineuse de la totalité de la zone de cornée à éliminer.

La présente invention a pour but d'éviter cet inconvénient, et concerne un système optique adapté pour conserver à un faisceau de lumière une quasi-symétrie de révolution dans l'espace image du système.

A cet effet, le système optique, destiné à être disposé entre une source susceptible d'émettre un faisceau de lumière et une cible, du type comportant une première optique de focalisation dudit faisceau de lumière, ledit faisceau présentant à l'entrée de ladite optique une section au moins sensiblement circulaire et, du fait de caractéristiques inhérentes au faisceau de lumière, présentant au foyer image de ladite première optique de focalisation une section de forme oblongue, est caractérisé en ce qu'il comprend, dans le sens de propagation dudit faisceau, en aval de ladite première optique :

— des premiers moyens disposés au foyer image de ladite première optique de focalisation et constitués par un diaphragme d'ouverture en forme d'une fente disposée transversalement à la direction de propagation du faisceau et dont la largeur varie entre zéro et la plus grande dimension de la section oblongue du faisceau à l'emplacement desdits premiers moyens ;

— des moyens de déplacement relatif entre ledit diaphragme et le restant dudit système transversalement à la direction de propagation du faisceau, de sorte que lesdits premiers moyens sont susceptibles de prélever une partie variable de l'aire de la section dudit faisceau audit foyer image ; et

— une seconde optique de focalisation, de sorte que, pour tout déplacement relatif de la cible et dudit système optique dans l'espace image du système, ladite aire prélevée par lesdits moyens est réglable de façon à obtenir, dans ledit espace au niveau de la cible, une section du faisceau qui reste au moins sensiblement circulaire.

Ainsi, quand ledit système optique est, par exemple, utilisé dans un appareil chirurgical destiné à modifier la courbure de la cornée par photodécomposition par un faisceau de lumière ultraviolette, la tache lumineuse créée sur l'oeil peut conserver, pendant toute la durée du traitement, c'est-à-dire quand la position relative de l'oeil et du système varie dans l'espace image du système, une forme circulaire, ou tout au moins, une forme quasiment circulaire dont l'ellipticité est maintenue dans une limite acceptable. Lesdits moyens de déplacement permettent de diaphragmer le faisceau selon une largeur réglable, inférieure ou égale à la plus grande dimension de la section oblongue du faisceau.

Certaines sources, notamment de lumière ultraviolette, émettant un faisceau de section au moins approximativement rectangulaire, dans ce cas, le système optique comprend, entre ladite source et ladite première optique de focalisation, des seconds moyens destinés à diaphragmer ledit faisceau, à la sortie de ladite source, suivant une section au moins sensiblement circulaire.

En particulier, lesdits seconds moyens peuvent être disposés au moins sensiblement au foyer objet de ladite première optique de focalisation.

Avantageusement, ladite seconde optique de focalisation est située, par rapport audits premiers moyens, à une distance égale à au moins sensiblement deux fois la distance focale de ladite seconde optique.

Avantageusement, la largeur de ladite fente varie de façon continue.

En particulier, lesdits moyens de déplacement sont associés audit diaphragme.

Selon encore une autre caractéristique de l'invention, lesdits seconds moyens sont constitués par un diaphragme présentant une ouverture circulaire de diamètre au moins sensiblement égal à la plus petite dimension de la section du faisceau à la sortie de la source.

Avantageusement, les première et seconde optiques de focalisation sont des lentilles ayant la même distance focale.

Les figures du dessin annexé feront bien comprendre comment l'invention peut être réalisée. Sur ces figures, des références identiques désignent des éléments semblables.

La figure 1 est le schéma synoptique d'ensemble d'un appareil chirurgical comportant le système optique de l'invention.

La figure 2 est une vue en perspective schématique du système optique de l'invention.

Les figures 3A à 3C illustrent différentes conditions de filtrage selon différentes positions relatives de la cible et du système optique.

Dans l'exemple de réalisation illustré, le système optique 1 fait partie d'un appareil chirurgical 2 destiné à modifier la courbure de la cornée.

Le système optique 1 est destiné à être disposé entre une source de lumière ultraviolette 3 et une cible, en l'occurrence un oeil 4.

La source lumineuse 3, dans cette application de l'invention à l'appareil chirurgical 2, est susceptible d'émettre un faisceau lumineux homogène 5 dont la longueur d'onde est voisine de 0,2 micromètre, par exemple un générateur laser à excimère, du type à mélange argon-fluor.

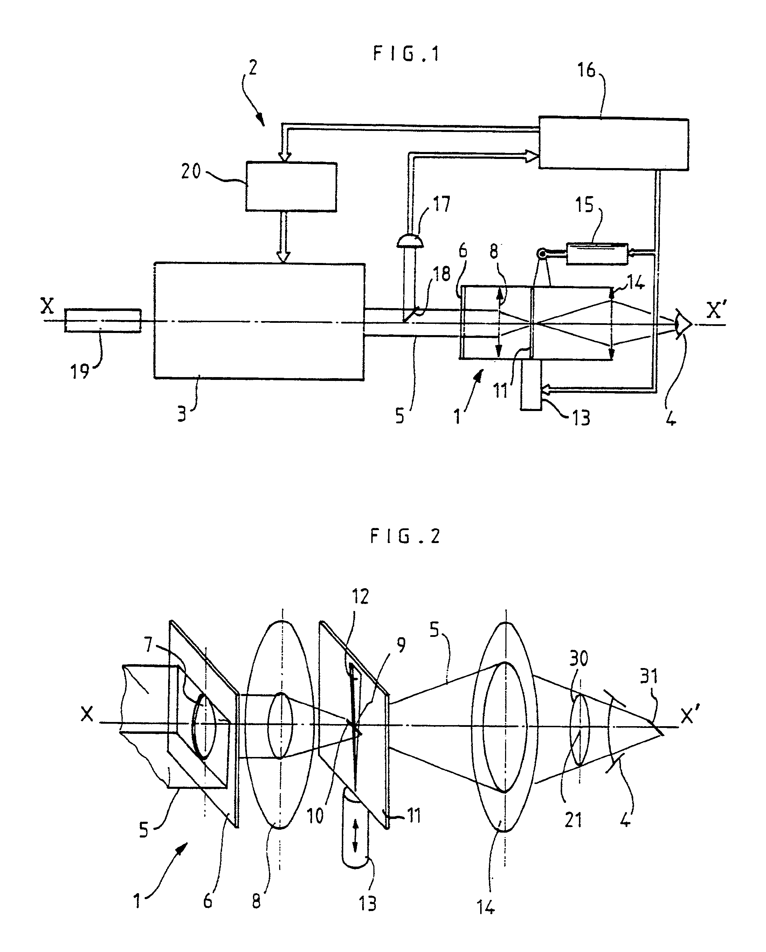

En se référant plus particulièrement à la figure 2, le système optique 1 comprend successivement, dans le sens de propagation du faisceau 5 (c'est-à-dire de la gauche vers la droite sur la figure 2) :

— un premier diaphragme 6 présentant une ouverture circulaire 7 de diamètre au moins sensiblement égal à la plus petite dimension de la section du faisceau 5 à la sortie de la source 3 (dans l'exemple illustré, la section du faisceau à la sortie de la source 3 étant rectangulaire, le diamètre de l'ouverture 7 sera égal à la largeur dudit faisceau 5) ;

— une première lentille 8 destinée à focaliser le faisceau de lumière 5, ledit faisceau présentant, grâce au diaphragme 6 qui peut être, par exemple, situé au foyer objet de la lentille 8, une section circulaire, et, du fait de caractéristiques inhérentes au faisceau de lumière, présentant au foyer image 9 de la lentille 8 une section de forme oblongue, ce qui peut être schématisé par la "barrette" 10 de la figure 2 ;

— un second diaphragme 11 dont l'ouverture 12 se présente sous forme d'une fente en V disposée transversalement à la direction de propagation X-X' du faisceau 5 et dont la largeur varie entre zéro et la longueur de la barrette 10, ledit diaphragme 11 étant disposé au foyer image 9 de la lentille 8 et pouvant ainsi prélevé une partie variable de l'aire de la section du faisceau 5 audit foyer image quand le diaphragme est déplacé transversalement à ladite direction X-X' à l'aide des moyens 13 ; et

— une seconde lentille 14 qui refocalise le faisceau lumineux en direction de la cible, et qui peut être située, par rapport au diaphragme 11, à une distance égale à deux fois la distance focale de la lentille 14, les lentilles 8 et 14 pouvant avoir la même distance focale.

Ainsi, pour tout déplacement relatif à l'aide de moyens 15 de l'oeil 4 et du système optique 1 dans l'espace image du système, ladite aire prélevée par le diaphragme 11 est réglable de façon à obtenir, sur l'oeil, une section du faisceau 5 qui reste au moins sensiblement circulaire, comme on le verra plus en détail par la suite.

L'appareil chirurgical 2 permet de modifier au moins en partie la courbure de la cornée de l'oeil 4 par ablation d'une zone de celle-ci en forme de lamelle lenticulaire d'épaisseur radialement variable, le faisceau de lumière ultraviolette de longueur d'onde voisine de 0,2 micromètre permettant la photodécomposition de la matière cornéenne, et le système optique 1 permettant de diriger ledit faisceau sur la zone de cornée à éliminer et formant une tache lumineuse sur une partie de ladite zone, ladite tache étant susceptible d'explorer la totalité de ladite zone de cornée.

En plus de la source lumineuse 3 et du système optique 1, l'appareil chirurgical 2 comprend :

— un calculateur électronique 16 chargé de piloter le processus de fonctionnement dudit appareil, et notamment de commander le système optique 1, plus spécialement les moyens de déplacement 13 du diaphragme 11 transversalement à la direction de propagation X-X' du faisceau 5 et les moyens 15 permettant de déplacer le système 1, ou une partie de celui-ci comme la lentille 14, par rapport à l'oeil 4 ;

— un photodétecteur 17, associé à un miroir semi-transparent 18, et destiné à fournir au calculateur 16 des informations relatives à l'énergie des impulsions du faisceau 5 ;

— un laser d'alignement 19, par exemple du type héliumnéon, permettant un positionnement correct du faisceau 5 sur la cornée ;

— un dispositif 20 de commande de la source 3, lui-même commandé par le calculateur 16 ; et

— un kératomètre automatique (non représenté) qui mesure en temps réel la courbure de la cornée et transmet ses mesures au calculateur, et une lampe à fente (non représentée) qui permet au chirurgien l'observation de la cornée en cours d'opération.

Le calculateur est programmé pour commander le système optique en fonction de la loi de variation d'épaisseur désirée pour la lamelle à éliminer de la cornée, et il exécute les séquences d'opérations correspondantes en prenant en compte les informations qui lui sont fournies par le kératomètre et le photodétecteur.

Grâce au système optique 1 de l'invention, on obtient, dans l'espace image du système (c'est-à-dire l'espace dans lequel se trouve physiquement l'oeil 4 sur lequel a lieu l'intervention) un faisceau dont la section évolue depuis une forme purement circulaire 30 au plan conjugué 21 du diaphragme 6 jusqu'à une forme 31 correspondant à celle de la section oblongue du faisceau 5 au foyer image 9 de la première lentille 8 en présentant ainsi une ellipticité de plus en plus accentuée qui est, en fait, corrigée, selon l'invention, grâce au diaphragme 11 à ouverture en V, qui est susceptible de prélever une aire variable de ladite section oblongue en maintenant ainsi, au niveau de la cible 4, l'ellipticité dans une limite prédéterminée.

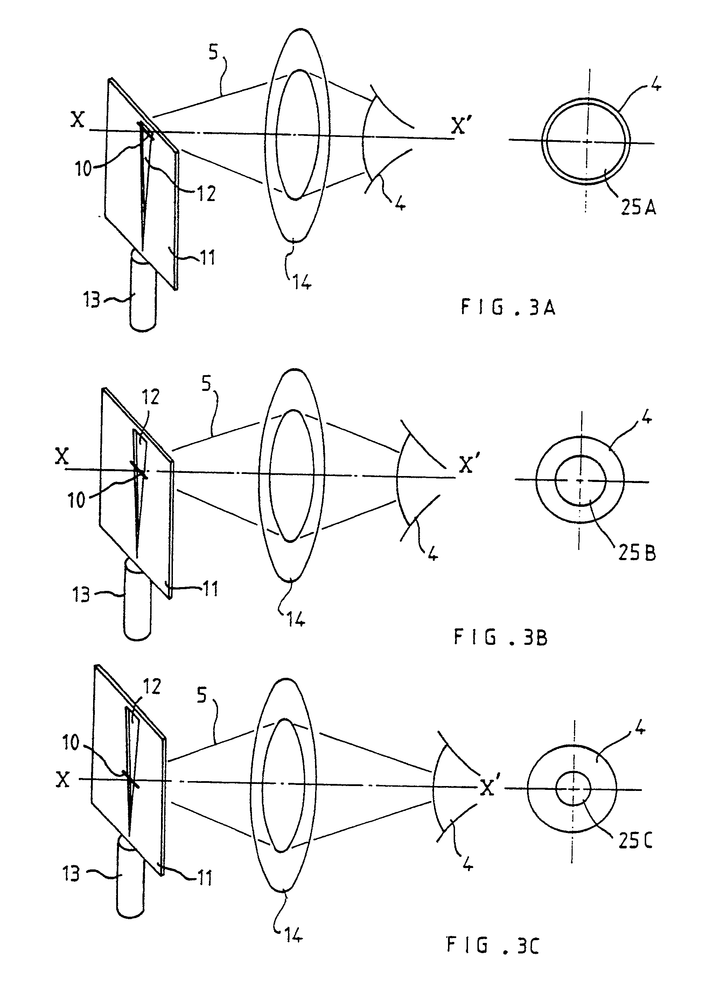

En d'autres termes, si la cible (l'oeil 4) est dans le plan conjugué du diaphragme 6 à travers l'ensemble du système optique, la section du faisceau 5 étant en ce point circulaire, le diaphragme 11 sera réglé transversalement à la direction de propagation X-X' du faisceau de façon qu'il prélève un maximum de ladite section oblongue, la zone d'ouverture maximale de la fente 12 étant alors en regard de ladite section oblongue ("barrette" 10) (figure 3A). On obtient alors, sur la cible 4, la tache lumineuse 25A montrée sur la partie droite (vue de face) de la figure 3A.

Quand la distance entre la seconde lentille 14 et la cible (l'oeil 4) augmente, la section du faisceau au niveau de la cible aurait tendance (sans diaphragme) à devenir de plus en plus allongée. Pour maintenir une section sensiblement circulaire, le diaphragme 11 sera réglé transversalement à la direction de propagation X-X' du faisceau de façon à prélever une portion d'aire de ladite section oblongue qui diminue quand la cible s'éloigne du plan conjugué du diaphragme 6 à travers l'ensemble du système optique 14 (et qui correspond aux taches lumineuses 25B et 25C des figures 3B et 3C).

En outre, ce dispositif maintient à un niveau sensiblement constant l'énergie lumineuse par impulsion et par unité de surface reçue par la cible, ce qui assure un processus de photoablation identique à lui-même pendant toute la durée de l'intervention.

Ainsi, le système optique de l'invention assure, à chaque instant, un filtrage permettant d'obtenir une puissance optique maximale compte tenu de la condition de maintien de la symétrie de révolution de la tache lumineuse sur la cible. Le temps global de l'intervention chirurgicale est ainsi minimisé, et les conditions de photodécomposition varient dans un intervalle plus faible.

## Revendications

1. Système optique, destiné à être disposé entre une source susceptible d'émettre un faisceau de lumière et une cible, du type comportant une première optique de focalisation dudit faisceau de lumière, ledit faisceau présentant à l'entrée de ladite optique une section au moins sensiblement circulaire et, du fait de caractéristiques inhérentes au faisceau de lumière, présentant au foyer image de ladite première optique de focalisation une section de forme oblongue, caractérisé en ce qu'il comprend, dans le sens de propagation dudit faisceau (5), en aval de ladite première optique (8) :

— des premiers moyens (11) disposés au foyer image (9) de ladite première optique (8) de focalisation et constitués par un diaphragme d'ouverture en forme d'une fente (12) disposée transversalement à la direction de propagation (X-X') du faisceau (5) et dont la largeur varie entre zéro et la plus grande dimension de la section oblongue (10) du faisceau (5) à l'emplacement desdits premiers moyens ;

— des moyens (13) de déplacement relatif entre ledit diaphragme et le restant dudit système transversalement à la direction de propagation (X-X') du faisceau (5), de sorte que lesdits premiers moyens (11) sont susceptibles de prélever une partie variable de l'aire de la section (10) dudit faisceau (5) dudit foyer image (9) ; et

— une seconde optique (14) de focalisation, de sorte que, pour tout déplacement relatif de la cible (4) et dudit système optique (1) dans l'espace image du système, ladite aire prélevée par lesdits moyens (11) est réglable de façon à obtenir, dans ledit espace au niveau de la cible (4), une section du faisceau (5) qui reste au moins sensiblement circulaire.

2. Système optique selon la revendication 1, caractérisé en ce qu'il comprend entre ladite source (3) qui émet un faisceau de section au moins approximativement rectangulaire et ladite première optique (8) de focalisation, des seconds moyens (6) destinés à diaphragmer ledit faisceau (5), à la sortie de ladite source (3), suivant une section au moins sensiblement circulaire.

3. Système optique selon la revendication 2, caractérisé en ce que lesdits seconds moyens (6) sont disposés au moins sensiblement au foyer objet de ladite première optique (8) de focalisation.

4. Système optique selon l'une quelconque des revendications 1 à 3, caractérisé en ce que ladite seconde optique (14) de focalisation est située, par rapport auxdits premiers moyens (11), à une distance égale à au moins sensiblement deux fois la distance focale de ladite seconde optique (14).

5. Système optique selon la revendication 1, caractérisé en ce que la largeur de ladite fente (12) varie de façon continue.

6. Système optique selon l'une quelconque des revendications 1 à 5, caractérisé en ce que lesdits moyens (13) de déplacement sont associés audit diaphragme (11).

7. Système optique selon l'une quelconque des revendications 2 à 6, caractérisé en ce que lesdits seconds moyens sont constitués par un diaphragme (6) présentant une ouverture circulaire (7) de diamètre au moins sensiblement égal à la plus petite dimension de la section du faisceau à la sortie de la source (3).

8. Système optique selon l'une quelconque des revendications 1 à 7, caractérisé en ce que les première (8) et seconde (14) optiques de focalisation sont des lentilles ayant la même distance focale.

9. Appareil chirurgical destiné à modifier au moins en partie la courbure de la cornée par ablation d'une zone de celle-ci en forme de lamelle lenticulaire d'épaisseur radialement variable, et comportant une source de lumière susceptible d'émettre un faisceau dont la longueur d'onde est voisine de 0,2 micromètre pour permettre la photodécomposition de la matière cornéenne, caractérisé en ce qu'il comprend un système optique selon l'une quelconque des revendications 1 à 8, ledit système optique étant adapté à diriger ledit faisceau sur ladite zone de cornée à éliminer et à former une tache lumineuse sur une partie de ladite zone, ladite tache étant susceptible d'explorer la totalité de ladite zone de cornée.

**Patentansprüche**

1. Optisches System zur Anordnung zwischen einer ein Lichtbündel aussendenden Quelle und einem Ziel, mit einer ersten Fokussierungsoptik des Lichtbündels, welches am Eingang der Optik einen wenigstens annähernd kreisförmigen Querschnitt hat und infolge von dem Lichtbündel inhärenten Eigenschaften in der bildseitigen Brennebene der ersten Fokussierungsoptik einen Querschnitt länglicher Form hat, dadurch gekennzeichnet, daß es in Ausbreitungsrichtung des Bündels (5) nach der ersten Optik (8) aufweist :

— erste in der bildseitigen Brennebene (9) der ersten Fokussierungsoptik (8) angeordnete Mittel (11), bestehend aus einer Blende mit einer Öffnung in Form eines quer zur Ausbreitungsrichtung (X-X') des Bündels (5) angeordneten Spaltes (12), dessen Breite zwischen null und der größten Dimension des länglichen Querschnitts (10) des Bündels (5) am Ort dieser Mittel variiert,

— Mittel (13) zur relativen Verstellung zwischen der Blende und dem Rest des Systems quer zur Ausbreitungsrichtung (X-X') des Bündels (5), und zwar in der Weise, daß die ersten Mittel (11) einen veränderbaren Teil der Fläche des Querschnitts (10) des Bündels (5) in der bildseitigen Brennebene (9) wegnehmen, und

— eine zweite Fokussierungsoptik (14), derart, daß für jede relative Verstellung des Ziels (4) und des optischen Systems (1) im Bildraum des Systems die durch die Mittel (11) weggenommene Fläche in der Weise regelbar ist, daß innerhalb des Bildraumes in der Ebene des Ziels (4) ein Querschnitt des Bündels (5) entsteht, der zumindest annähernd kreisförmig bleibt.

2. Optisches System nach Anspruch 1, dadurch gekennzeichnet, daß es zwischen der Quelle (3), die ein Bündel von wenigstens annähernd rechteckigem Querschnitt aussendet, und der ersten Fokussierungsoptik (8) zweite Mittel (6) aufweist, die dazu bestimmt sind, das Bündel (5) am Ausgang der Quelle (3) auf einen wenigstens annähernd kreisförmigen Querschnitt abzublenden.

3. Optisches System nach Anspruch 2, dadurch gekennzeichnet, daß die zweiten Mettel (6) wenigstens annähernd in der objektseitigen Brennebene der ersten Fokussierungsoptik (8) angeordnet sind.

4. Optisches System nach einem der vorhergenden Ansprüche 1 bis 3, dadurch gekennzeichnet, daß die zweite Fokussierungsoptik (14) von den ersten Mitteln (11) einen Abstand hat, der wenigstens annähernd gleich der doppelten Brennweite der zweiten Optik (14) ist.

5. Optisches System nach Anspruch 1, dadurch gekennzeichnet, daß die Breite des Spalts (12) sich kontinuierlich ändert.

6. Optisches System nach einem der vorhergehenden Ansprüche 1 bis 5, dadurch gekennzeichnet, daß die Verstellmittel (13) mit der Blende (11) verbunden sind.

7. Optisches System nach einem der vorhergehenden Ansprüche 2 bis 6, dadurch gekennzeichnet, daß die zweiten Mettel aus einer Blende (6) bestehen, die eine kreisförmige Öffnung (7) aufweist, deren Durchmesser wenigstens annähernd so groß ist wie die kleinste Abmessung des Querschnitts des Bündels am Ausgang der Quelle (3).

8. Optisches System nach einem der vorhergehenden Ansprüche 1 bis 7, dadurch gekennzeichnet, daß die erste (8) und zweite (14) Fokussierungsoptik Linsen gleicher Brennweite sind.

9. Chirurgisches Gerät zur wenigstens teilweisen Modifizierung der Krümmung der Hornhaut durch Wegnahme einer Zone derselben in Form einer linsenförmigen Lamelle von radial variabler Dicke, und mit einer Lichtquelle, die ein Bündel aussendet, dessen Wellenlänge etwa 0,2 µ beträgt, um die Fotodekomposition der Hornhautmaterie zu ermöglichen, dadurch gekennzeichnet, daß es ein optisches System nach einem der Ansprüche 1 bis 8 aufweist, das dazu geeignet ist, das Bündel auf die zu entfernende Zone der Hornhaut zu richten und einen Lichtfleck auf einem Teil der Zone zu erzeugen, welcher Lichtfleck imstande ist, die gesamte Zone der Hornhaut zu explorieren.

## Claims

1. Optical system, adapted to be disposed between a source capable of emitting a beam of light and a target, of the type comprising first optics for focussing said beam of light, said beam presenting at the inlet of said optics an at least substantially circular section and, due to characteristics inherent in the beam of light, presenting at the image focus of said first focusing optics a section of oblong shape, characterized in that it comprises, in the direction of propagation of said beam (5), downstream of said first optics (8) :

— first means (11) disposed in the image focus (9) of said first focussing optics (8) and constituted by a diaphragm of which the opening is in the form of a slot (12) disposed transversely to the direction of propagation (X-X') of the beam (5) and of which the width varies between zero and the largest dimension of the oblong section (10) of the beam (5) at the location of said first means;

— means (13) for relative displacement between said diaphragm and the rest of said system transversely to the direction of propagation (X-X') of the beam (5), so that said first means (11) are capable of removing a variable part of the area of the section (10) of said beam (5) in said image focus (9) ; and

— second focussing optics (14) with the result that, for any relative displacement of the target (4) and said optical system (1) in the image space of the system, said area removed by said means (11) is adjustable so as to obtain, in said space at the level of the target (4), a section of beam (5) which remains at least substantially circular.

2. Optical system according to claim 1, characterized in that it comprises, between said source (3) which emits a beam of at least substantially rectangular section and said first focussing optics (8), second means (6) for diaphragming said beam (5), on leaving said source (3), along an at least substantially circular section.

3. Optical system according to claim 2, characterized in that said second means (6) are disposed at least substantially in the object focus of said first focussing optics (8).

4. Optical system according to anyone of claims 1 to 3, characterized in that said second focussing optics (14) is located, with respect to said first means (11), at a distance equal to at least substantially twice the focal distance of said second optics (14).

5. Optical system according to claim 1, characterized in that the width of said slot (12) varies continuously.

6. Optical system according to anyone of claims 1 to 5, characterized in that said displacement means (13) are associated with said diaphragm.

7. Optical system according to anyone of claims 2 to 6, characterized in that said second means are constituted by a diaphragm (6) presenting a circular opening (7) of diameter at least substantially equal to the smallest dimension of the section of the beam leaving the source (3).

8. Optical system according to anyone of claims 1 to 7, characterized in that the first (8) and second (14) focussing optics are lenses having the same focal distance.

9. Surgical apparatus for modifying at least partly the curvature of the cornea by ablation of a zone thereof in the form of a lenticular plate of radially variable thickness, and comprising a source of light capable of emitting a beam whose wave length is close to 0.2 micrometer to allow photodecomposition of the corneal matter, characterized in that it comprises an optical system according to anyone of claims 1 to 8, said optical system directing said beam onto said zone of cornea to be eliminated and forming a light spot on a part of said zone, said spot being capable of scanning the whole of said cornea zone.

FIG.1

FIG.2

FIG.3A

FIG.3B

FIG.3C